# EUROPEAN PATENT SPECIFICATION

(11) **EP 0 812 206 B1**
(45) Date of publication and mention of the grant of the patent: **10.07.2002**
(21) Application number: 96909555.3
(22) Date of filing: 29.02.1996
(51) Int. Cl.: A61K 38/17, A61K 39/395

(54) **CD40 BINDING PROTEIN FOR STIMULATING AN IMMUNE RESPONSE**
CD40 BINDENDES PROTEIN ZUR STIMULIERUNG DER IMMUNANTWORT
PROTEINE LIANT LE CD40 POUR STIMULER UNE REPONSE IMMUNITAIRE

(30) Priority: 01.03.1995 US 396230
(43) Date of publication of application: 17.12.1997
(73) Proprietor: IMMUNEX CORPORATION, Seattle Washington 98101 (US)
(72) Inventor: ALDERSON, Mark, Bainbridge Island, WA 98110 (US); CAMPBELL, Kim A., Dublin, Ohio 43107 (US); KENNEDY, Mary K., Seattle, WA 98117 (US); MALISZEWSKI, Charles R., Seattle, WA 98177 (US)
(74) Representative: Cornish, Kristina Victoria Joy
(86) International application number: US9602839
(87) International publication number: WO9626735

(56) References cited:
- WO-A-93/08207
- WO-A-94/04570
- WO-A-95/09653
- EUROPEAN JOURNAL OF IMMUNOLOGY 24 (1). 1994. 116-123, XP000573801 KENNEDY M K ET AL: "Induction of B cell costimulatory function by recombinant murine CD40 ligand."
- EUROPEAN JOURNAL OF IMMUNOLOGY 26 (2). 1996. 370-378, XP000573804 KENNEDY M K ET AL: "CD40 - CD40 ligand interactions are required for T cell-dependent production of interleukin-12 by mouse macrophages."
- 37TH ANNUAL MEETING OF THE AMERICAN SOCIETY OF HEMATOLOGY, SEATTLE, WASHINGTON, USA, DECEMBER 1-5, 1995. BLOOD 86 (10 SUPPL. 1). 1995. 596A, XP002006293 FANSLOW W C ET AL: "Administration of purified recombinant trimeric CD40 ligand augments humoral and cell-mediated immune responsiveness in CD40L -deficient mice."
- CURRENT OPINION IN IMMUNOLOGY 7 (2). 1995. 243-247, XP002006294 ARMITAGE R J ET AL: "B-cell stimulation."

## Description

### TECHNICAL FIELD OF THE INVENTION

The present invention relates to a method of stimulating an immune response in an individual, comprising administering an effective amount of an oligomeric CD40 ligand, and compositions useful therefor.

### BACKGROUND OF THE INVENTION

An immune response to a pathogen can be classified broadly as either being cell-mediated (cellular immunity) or antibody mediated (humoral immunity). In cellular immunity, activated macrophages and cytotoxic lymphocytes carry out elimination of the pathogen. Humoral immunity, in contrast, operates primarily through antibody production. It is currently believed that these two arms of the immune response are regulated by distinct subsets of helper T (T_{H}) cells which secrete specific arrays of cytokines (reviewed in *Immunological Reviews* 123, 1991).

Type 1 T_{H} cells (T_{H}1 cells) mediate delayed type hypersensitivity (DTH), and secrete Interferon-γ (IFN-γ) and Interleukin-2 (IL-2), while Type 2 T_{H} cells (T_{H}2 cells) secrete primarily Interleukins 4, 5 and 10 (IL-4, IL-5 and IL-10, respectively) and provide B cell help. Development of the immune response along either T_{H}1 or T_{H}2 pathway is often apparent early in an infection, and appears to be governed by the type of organism causing the infection (Scott and Kaufmann, *Immunol. Today* 12:346, 1991), and by the genetic makeup of the infected host. Failure to resolve disease or development of immunopathology can result when the immune response proceeds inappropriately. The ability to manipulate an immune response toward either a T_{H}1, cell mediated response, or T_{H}2, antibody mediated response, would provide a useful tool not only in infectious disease, but in inflammatory and allergic diseases as well (see, for example, Powrie and Coffman, *Immunol. Today* 14:270, 1993).

### SUMMARY OF THE INVENTION

The present invention relates to the manufacture of a composition for a method of treating a mammal infected with a pathogenic or opportunistic organism, which composition comprises a CD40 binding protein effective to stimulate a protective T_{H}1 immune response against the organism. CD40 binding proteins are pharmaceutical compositions capable of binding CD40 and transducing a biological signal. Pathogenic or opportunistic organisms include, for example, species of *Leishmania, Listeria, Mycobacteria, Salmonella,* *Trypanosoma*, *Pneumocystis*, and *Toxoplasma,* individually or in combination. CD40 binding proteins are selected from the group consisting of CD40 ligand, monoclonal antibodies that specifically bind CD40, and combinations thereof.

Interleukin-12 (IL-12) is a key cytokine, produced by macrophages/monocytes, which is crucial in influencing the development of a protective T_{H}1 response. IL-12 is produced upon stimulation with CD40 binding proteins. CD40 binding proteins will thus be useful in treating disease for which a T_{H}1 response is desirable, in prevention of disease through development of an appropriate T_{H}1 response, and in treatment of disease in individuals who have depressed cell mediated immunity.

### BRIEF DESCRIPTION OF THE DRAWINGS

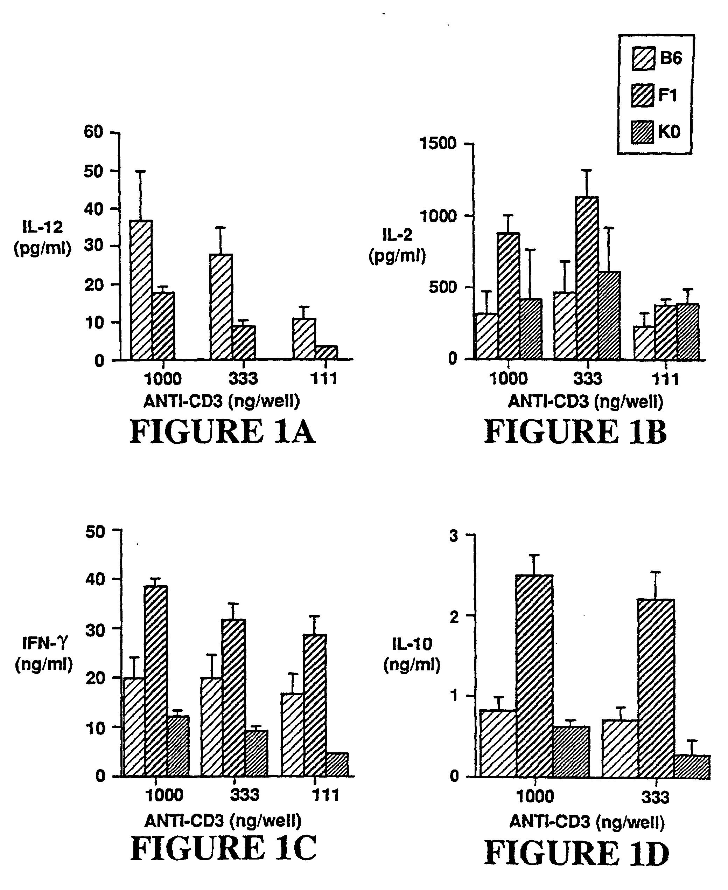

Figure 1 demonstrates that anti-CD3 activated splenocytes from CD40L deficient mice are impaired in their ability to produce IL-12, but not IL-2, IFN-γ or IL-10. Splenocytes were treated as described in Example 3, and supernatants were tested by bioassay for the presence of IL-12 or IL-2 (Figures 1A and 1B), or by enzyme immunoassay for IFN-γ or IL-10 (Figures 1C and 1D).

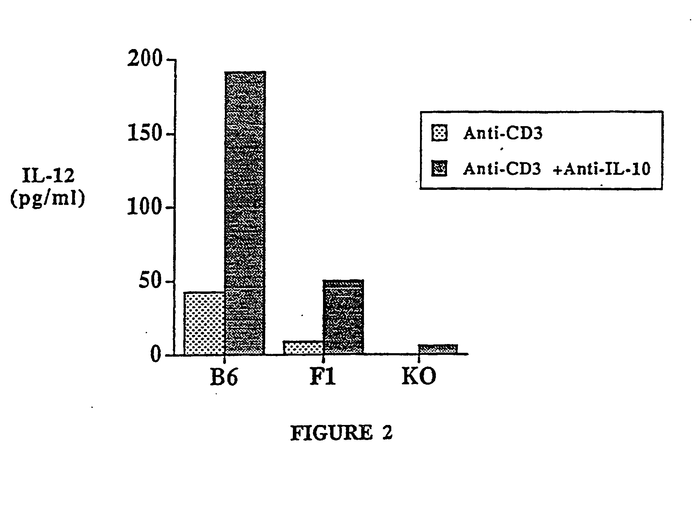

Figure 2 illustrates enhancement of T cell-dependent IL-12 production by anti-IL-10. Assays were performed as described in Example 4. Supernatant from control splenocytes stimulated with anti-CD3 in the presence of anti-IL-10 contained approximately four-fold higher levels of IL-12 than those generated in the absence of anti-IL-10. Furthermore, low levels of IL-12 were detected in supernatants for CD40L KO splenocytes incubated with both anti-CD3 and anti-IL-10.

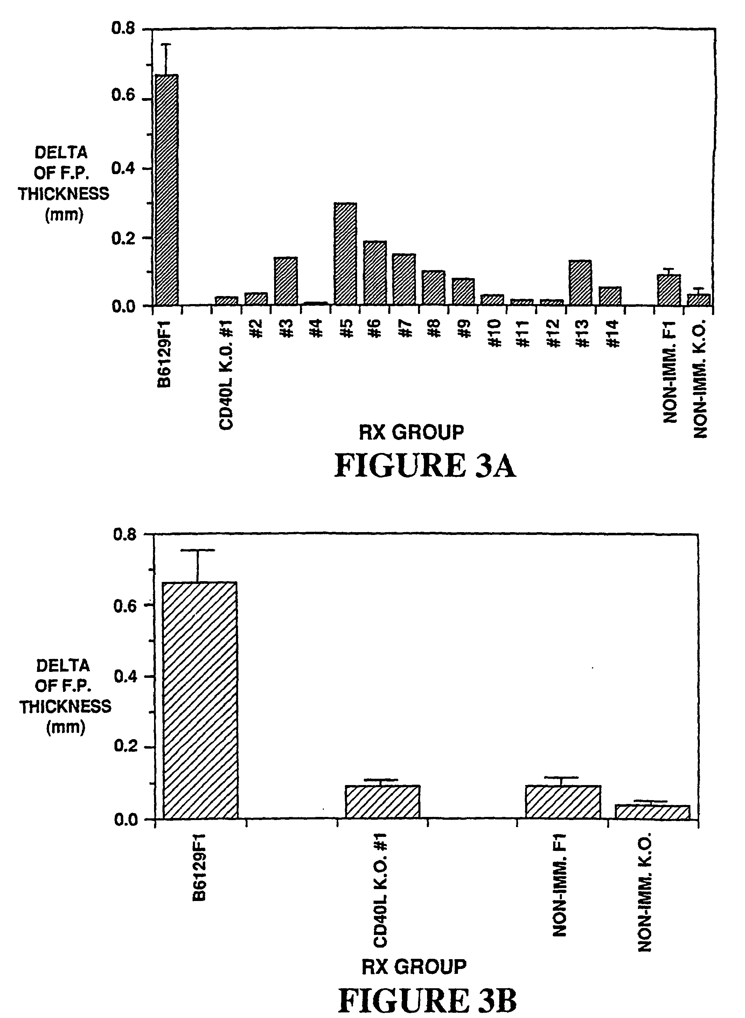

Figure 3 presents results that confirm that CD40L is critical for the development of a cellular immune response to antigen. The experiments were conducted as described in Example 6. Although there was some variation in DTH response among the individual CD40L KO mice tested (Figure 3A), as a group, CD40L-deficient mice are severely impaired in their ability to mount a DTH response to antigen (Figure 3B).

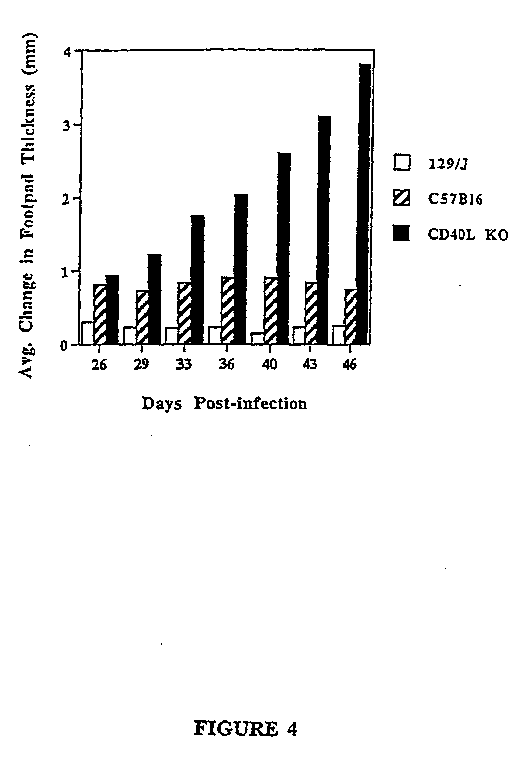

Figure 4 shows the progressive swelling of the footpad subsequent to infection with *L. major* observed in CD40 ligand deficient mice, in contrast to the parental mouse strains from which they were derived. Mice were infected as described in Example 7, and disease progression determined by measuring footpad thickness.

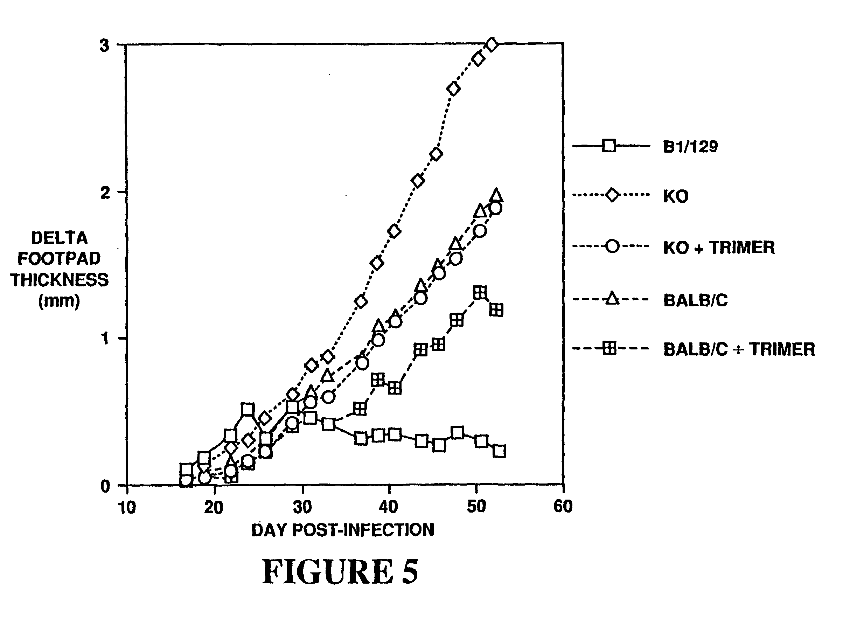

Figure 5 presents results that indicate that administration of CD40LT decreases the severity of infection in susceptible mice. Infected mice were treated as described in Example 8, and the effect of treatment determined by measuring footpad thickness.

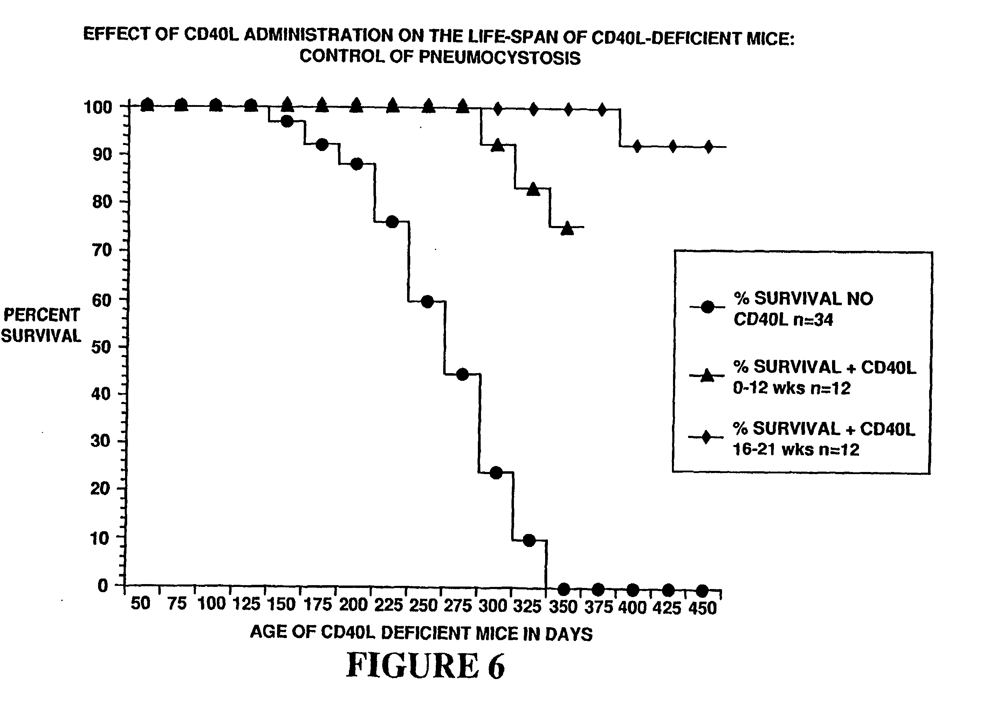

Figure 6 demonstrates that CD40LT controls or ameliorates *Pneumocystis* infection in susceptible, CD40 ligand deficient mice. The mice were treated as described in Example 9, and necropsied to determine cause of death.

### DETAILED DESCRIPTION OF THE INVENTION

The present invention relates to the manufacture of a composition for a method of treating a mammal infected with a pathogenic or opportunistic organism, which composition comprises an amount of a CD40 binding protein that is effective at stimulating an immune response against the pathogen. Mice infected with the pathogen *Leishmania* were treated with a CD40 binding protein, which stimulated an effective immune response against the pathogen. The murine model used is believed by those skilled in the art to provide results that are correlatable to results obtained in various mammalian species, including humans, and that also pertain to other infectious organisms.

Thus, the findings described herein, in a predictive animal model, provide data to fully enable the inventive use in method of treating a mammal infected with a pathogenic or opportunistic organism, of a pharmaceutical composition comprising a substance with CD40 binding protein activity.

### Pathogenic/Opportunistic Organisms

Pathogenic organisms are organisms that are capable of causing disease in a healthy individual, whereas opportunistic organisms usually do not cause disease in a healthy individual, but may result in disease conditions in immunocompromised hosts. Both types of organisms include viruses, bacteria, yeast, fungi, and protozoa. Additionally, in some syndromes, multiple organisms may be present, and may play a causative role in the syndrome.

An exemplary pathogenic protozoan is *Leishmania,* an obligate intracellular macrophage parasite that causes a variety of diseases characterized by visceral, cutaneous, or mucosal lesions. Different species and isolates of *Leishmania* vary in their ability to infect and replicate in macrophages both *in vivo* and *in vitro.* Clinically, infections with *L. braziliensis* present as single or multiple cutaneous lesions, with a small percentage progressing to a more severe mucosal disease. While the cutaneous lesions may heal spontaneously or respond well to chemotherapy, mucosal lesions are often highly destructive and relatively refractory to treatment. Even if the mucosal lesion cures, there is often spontaneous relapse, perhaps years later. The course of infection with the protozoa and macrophage pathogen *Leishmania* is determined, in part, by their early replication in macrophages, the exclusive host cells for these organisms. Although factors contributing to the inhibition or proliferation of *Leishmania* are not well understood, certain cytokines can influence the course of infection. For example, IL-12 cures leishmaniasis in susceptible BALB/c mice (Heinzel et al., *J. Exp. Med.* 177:1505, 1993).

The pathogenic hemoflagellate protozoan *Trypanosoma cruzi* (*T. cruzi*) causes Chagas' disease, a major public health problem in many countries of Latin America. Infection with this parasite may be acute or chronic, and frequently involves development of progressive pathology in tissues of the heart, esophagus and colon. The parasites infect a variety of nucleated cells, including macrophages. In both human and laboratory animals, *T. cruzi* infection is accompanied by a non-specific immune-suppression mediated by T cells and macrophages. Mechanisms which control parasite replication during the acute and chronic phases and which maintain low but persistent numbers of circulating parasites during the chronic phase are not well understood.

Additional examples of pathogens include *Mycobacterium tuberculosis* and *Mycobacterium leprae,* as well as the protozoan *Toxoplasma gondii*. The fungi *Histoplasma capsulatum, Candida albicans*, *Candida parapsilosis,* and *Cryptococcus neoformans* can also be considered opportunistic or pathogenic organisms. Certain of the Rickettsia, for example, *R. prowazekii, R. coronii,* and *R. tsutsugamushi* are also included, as are combinations of two or more organisms.

In addition to infecting humans, many of these organisms infect other mammals, which then can serve as a reservoir of infection for humans. For example, domesticated dogs are believed to serve as a major reservoir of *Leishmania*, while cats are known to carry *Toxoplasma.* Methods of augmenting a mammals' immune and/or inflammatory response against these organisms are thus likely to be useful in species of mammals other than humans.

### CD40

Human CD40 antigen (CD40) is a peptide of 277 amino acids having a molecular weight of 30,600 (Stamenkovic et al., *EMBO J.* 8:1403, 1989). A cDNA encoding human CD40 was isolated from a cDNA library prepared from Burkitt lymphoma cell line Raji. The putative protein encoded by the CD40 cDNA contains a putative leader sequence, trans-membrane domain and a number of other features common to membrane-bound receptor proteins. CD40 has been found to be expressed on B lymphocytes, epithelial cells and some carcinoma cell lines.

CD40 is a member of the tumor necrosis factor (TNF)/nerve growth factor (NGF) receptor family, which is defined by the presence of cysteine-rich motifs in the extracellular region (Smith et al., *Science* 248:1019, 1990; Mallett and Barclay, *Immunology Today* 12:220; 1991). This family includes the lymphocyte antigen CD27, CD30 (an antigen found on Hodgkin's lymphoma and Reed-Sternberg cells), two receptors for TNF, a murine protein referred to as 4-1BB, rat OX40 antigen, NGF receptor, and *Fas* antigen.

CD40 may be detected on the surface of a cell by any one of several means known in the art. For example, an antibody specific for CD40 may be used in a fluorescence-activated cell sorting technique to determine whether cells express CD40. Other methods of detecting cell surface molecules are also useful in detecting CD40.

### CD40 Monoclonal Antibodies

Monoclonal antibodies directed against the CD40 surface antigen (CD40 mAb) have been shown to mediate various biological activities on human B cells. For example, CD40 mAb induce homotypic and heterotypic adhesion (Barrett et al., *J. Immunol. 146*:1722*,* 1991; Gordon et al., *J. Immunol. 140*:1425, 1988), and increase cell size (Gordon et al., *J. Immunol. 140*:1425, 1988; Valle et al., *Eur. J. Immunol. 19*:1463, 1989). CD40 mAb also induce proliferation of B cells activated with anti-IgM, CD20 mAb, or phorbol ester alone (Clark and Ledbetter, *Proc. Natl. Acad. Sci. USA 83*:4494, 1986; Gordon et al., LEUKOCYTE TYPING III; A.J. McMichael ed. Oxford University Press. Oxford, p. 426; Paulie et al., *J. Immunol. 142*:590, 1989) or in concert with IL-4 (Valle et al., *Eur. J. Immunol. 19*:1463, 1989; Gordon et al., *Eur. J. Immunol. 17*:1535, 1987), and produce IgE (Jabara et al., *J. Exp. Med*. *172*:1861, 1990; Gascan et al., *J. Immunol. 147*:8, 1991), IgG, and IgM (Gascan et al., *J. Immunol. 147*:8, 1991) from IL-4-stimulated T cell-depleted cultures.

In addition, CD40 mAb have been reported to enhance IL-4-mediated soluble CD23/FcεRII release from B cells (Gordon and Guy, *Immunol. Today 8*:339, 1987; Cairns et al., *Eur. J. Immunol. 18*:349, 1988) and to promote B cell production of IL-6 (Clark and Shu, *J. Immunol. 145*:1400, 1990). Recently, in the presence of CDw32+ adherent cells, human B cell lines have been generated from primary B cell populations with IL-4 and CD40 mAb (Banchereau et al., *Science 241*:70, 1991). Furthermore, germinal center centrocytes can be prevented from undergoing apoptosis if they are activated through CD40 and/or receptors for antigen (Liu et al., *Nature 342*:929, 1989). Each of the above publications describes CD40 mAb that stimulate a biological activity of B cells.

U.S.S.N. 08/130, 541, filed October 1, 1993, the relevant disclosure of which is incorporated by reference, discloses two monoclonal antibodies that specifically bind CD40, referred to as hCD40m2 and hCD40m3. Unlike other CD40 mAb, hCD40m2 (ATCC HB11459) and hCD40m3 bind CD40 and inhibit binding of CD40 to cells that constitutively express CD40L. Greater than 95% inhibition of binding was observed with hCD40m2 or with CD40 mAb M3, at concentrations as low as 12.5µg/ml, as compared to irrelevant IgG or a control CD40 mAb, G28.5. hCD40m2 was also able to inhibit CD40L-induced TNF-α production.

Additional CD40 monoclonal antibodies may be generated using conventional techniques (*see* U.S. Patent Nos. RE 32,011, 4,902,614, 4,543,439, and 4,411,993 which are incorporated herein by reference; *see also Monoclonal Antibodies,* *Hybridomas: A New Dimension in Biological Analyses,* Plenum Press, Kennett, McKearn, and Bechtol (eds.), 1980, and *Antibodies: A Laboratory Manual,* Harlow and Lane (eds.), Cold Spring Harbor Laboratory Press, 1988, which are also incorporated herein by reference).

Briefly, an animal is injected with a form of CD40 suitable for generating an immune response against CD40. The animal may be reimmunized as needed until levels of serum antibody to CD40 have reached a plateau, then be given a final boost of soluble CD40, and three to four days later sacrificed. Organs which contain large numbers of B cells such as the spleen and lymph nodes are harvested and disrupted into a single cell suspension by passing the organs through a mesh screen or by rupturing the spleen or lymph node membranes which encapsulate the cells.

Alternatively, suitable cells for preparing monoclonal antibodies are obtained through the use of *in vitro* immunization techniques. Briefly, an animal is sacrificed and the spleen and lymph node cells are removed. A single cell suspension is prepared, and the cells are placed into a culture which contains a form of CD40, which is suitable for generating an immune response as described above. Subsequently, the lymphocytes are harvested and fused as described below.

Cells which are obtained through the use of *in vitro* immunization or from an immunized animal as described above may be immortalized by transfection with a virus. For example, the Epstein bar virus (EBV; *see* Glasky and Reading, *Hybridoma 8(4)*:377-389, 1989) can transform human B cells. Alternatively, the harvested spleen and/or lymph node cell suspensions are fused with a suitable myeloma cell in order to create a "hybridoma" which secretes monoclonal antibody. Suitable myeloma lines are preferably defective in the construction or expression of antibodies, and are additionally syngeneic with the cells from the immunized animal. Many such myeloma cell lines are well known in the art and may be obtained from sources such as the American Type Culture Collection (ATCC), Rockville, Maryland *(see Catalogue of Cell Lines & Hybridomas,* 6th ed., ATCC, 1988).

### CD40 Ligand

Activated CD4+ T cells express high levels of a ligand for CD40 (CD40L). Human CD40L, a membrane-bound glycoprotein, has recently been cloned from peripheral blood T-cells as described in Spriggs et al., *J. Exp. Med.* 176:1543 (1992), and in United States Patent Application number 07/969,703, filed October 23, 1992, the disclosure of which is incorporated by reference herein. The cloning of murine CD40L is described in Armitage et al., *Nature* 357:80, 1992. CD40L induces B-cell proliferation in the absence of any co-stimulus, and can also induce production of immunoglobulins in the presence of cytokines. In addition, CD40 ligand-transfected cells can stimulate monocytes to become tumoricidal (Alderson et al., *J. Exp. Med.* 178:669, 1993).

CD40L is a type II membrane polypeptide having an extracellular region at its C-terminus, a transmembrane region and an intracellular region at its N-terminus. Soluble CD40L comprises an extracellular region of CD40L (amino acid 47 to amino acid 261 of SEQ ID NO:1) or a fragment thereof. CD40L biological activity is mediated by binding of the extracellular region of CD40L with CD40, and includes B cell proliferation and induction of antibody secretion (including IgE secretion).

U.S.S.N. 07/969,703 describes preparation of a soluble CD40L/Fc fusion protein referred to as CD40L/FC2. CD40L/FC2 contains an eight amino acid hydrophilic sequence described by Hopp et al. (Hopp et al., *Bio*/*Technology* 6:1204,1988; referred to as Flag®), an IgG₁ Fc domain, a linker sequence (described in U.S. Patent 5,073,627), and the extracellular region of human CD40L. Also described in U.S.S.N. 07/969,703 is a soluble CD40L fusion protein referred to as trimeric CD40L., which contains a 33 amino acid sequence referred to as a "leucine zipper," the eight amino acid hydrophilic sequence described by Hopp et al. *(supra),* followed by the extracellular region of human CD40L. Both oligomeric forms of CD40L induce human B cell proliferation in the absence of any co-stimuli, and (in conjunction with the appropriate cytokine) result in the production of IgG, IgE, IgA and IgM.

The CD40L/FC2 and the trimeric CD40L described in U.S.S.N. 07/969,703 will be useful in the present inventive methods, as will other forms of CD40L that can be prepared using known methods of preparing recombinant proteins.

### Additional CD40 Binding Proteins

Binding proteins may also be constructed utilizing recombinant DNA techniques to incorporate the variable regions of a gene which encodes an antibody to CD40. (*see* James W. Larrick et al., "Polymerase Chain Reaction Using Mixed Primers: Cloning of Human Monoclonal Antibody Variable Region Genes From Single Hybridoma Cells," *Biotechnology 7*:934-938, September 1989; Reichmann et al., "Reshaping Human Antibodies for Therapy," *Nature 332*:323-327, 1988; Roberts et al., "Generation of an Antibody with Enhanced Affinity and Specificity for its Antigen by Protein Engineering," *Nature 328*:731-734, 1987; Verhoeyen et al., "Reshaping Human Antibodies: Grafting an Antilysozyme Activity," *Science 239*:1534-1536, 1988; Chaudhary et al., "A Recombinant Immunotoxin Consisting of Two Antibody Variable Domains Fused to *Pseudomonas* Exotoxin," *Nature 339*:394-397, 1989).

Briefly, DNA encoding the antigen-binding site (or CD40 binding domain; variable region) of a CD40 mAb is isolated, amplified, and linked to DNA encoding another protein, for example a human IgG *(see* Verhoeyen et al., *supra; see also* Reichmann et al., *supra*). Alternatively, the antigen-binding site (variable region) may be either linked to, or inserted into, another completely different protein (*see* Chaudhary et al., *supra),* resulting in a new protein with antigen-binding sites of the antibody as well as the functional activity of the completely different protein.

Furthermore, DNA sequences which encode smaller portions of the antibody or variable regions which specifically bind to mammalian CD40 may also be utilized within the context of the present invention. Similarly, the CD40 binding region (extracellular domain) of a CD40 ligand may be used to prepare other CD40 binding proteins. DNA sequences that encode proteins or peptides that form oligomers will be particularly useful in preparation of CD40 binding proteins comprising an antigen binding domain of CD40 antibody, or an extracellular domain of a CD40 ligand. Certain of such oligomer-forming proteins are disclosed in U.S.S.N. 07/969,703; additional, useful oligomer-forming proteins are also disclosed in U.S.S.N. 08/107,353, filed August 13, 1993, and in U.S.S.N. 08/145,830, filed September 29, 1993.

Once suitable antibodies or binding proteins have been obtained, they may be isolated or purified by many techniques well known to those of ordinary skill in the art *(see Antibodies: A Laboratory Manual,* Harlow and Lane (eds.), Cold Spring Harbor Laboratory Press, 1988). Suitable techniques include peptide or protein affinity columns, HPLC or RP-HPLC, purification on protein A or protein G columns, or any combination of these techniques. Recombinant CD40 binding proteins can be prepared according to standard methods, and tested for binding specificity to the CD40 utilizing assays known in the art, including for example ELISA, ABC, or dot blot assays, as well by bioactivity assays such as those described for CD40 mAb.

### In vitro and in vivo models

The murine model of leishmaniasis described herein is recognized as an appropriate animal model of infectious disease requiring a T_{H}1 response. Murine models of many other infectious human diseases are known in the art. For example, Sher (*Imm. Rev.* 127:183-204, 1992), discusses murine models of several different human diseases, including acquired immunodeficiency syndrome (AIDS), toxoplasmosis, leishmaniasis, trypanosomiasis, and shistosomiasis. Nathan (in: *Mechanisms of Host Resistance to Infectious Agents, Tumors*, *and Allografts*, R.M. Steinman and R.J. North, eds., Rockefeller University Press, New York, pp.165-184, 1986) also reviews the use of mice in the study of various human diseases, and further presents results of studies performed in humans that confirm results first observed in murine models. Rats and/or mice have also been used in animal models of cryptosporidiosis (Meulbroek et al., *Workshop on Pneumocystis, Cryptospridium and Microsporidium* 113S), *Salmonella typhimurium* infections (Hougen et al., *APMIS* 98:30; 1990), Mycobacterium avium infections (Furney et al., *Antimicrobial Agents and Chemotherapy* 34:1629; 1990), and of *Pneumocystis carinii* pneumonia (Boylan and Current, *J. Protozool.* 38:138S; 1991; Soulez et al., *Workshop on Pneumocystis, Cryptospridium and Microsporidium* 123S)

Other species also provide useful animal models. For example, Wyand *(AIDS Res. and Human Retroviruses* 8:349; 1992) discusses the use of SIV-infected Rhesus monkeys for the preclinical evaluation of AIDS drugs and vaccines. Simian and feline models (Gardner, *Antiviral Res.* 15:267; 1991; Stahl-Hennig et al., *AIDS* 4:611; 1990) and murine models (Ruprecht et al.. *Cancer Res.* 50:5618s; 1990) have been proposed for evaluating anti-retroviral therapy. Rhesus monkeys have also been used in a model of Chagas' disease (Bonecini-Almeida et al., *Mem. Inst. Osaldo Cruz* 85:163; 1990; Rio de Janeiro). Various non-human primates have been observed to suffer naturally- or experimentally-acquired leprosy (Meyers et al., *Am. J. Trop. Med and Hyg.* 44:24; 1991). Those skilled in the art recognize these and many other possible animal models of disease caused by macrophage pathogens.

### Macrophages/monocytes

Activated macrophages ingest (phagocytose) microbes, produce and release highly reactive intracellular oxygen species, and secrete various cytokines that upregulate immune and inflammatory responses of the mammal to the microbe or microbes. Activation of macrophages is confirmed *in vitro* by various means involving measuring one or more of these activities.

One of the primary functions of peripheral blood monocytes is to regulate an immune or inflammatory response by synthesis and secretion of an array of biologically active molecules including enzymes, plasma proteins and cytokines. Monocyte-derived cytokines include IL-1α, IL-1β, IL-6, IL-8, IL-12, and TNF-α. All of these cytokines, produced by monocytes, have broad immunoregulatory properties that are central to the host response to infection.

Microbial products, such as LPS and peptidoglycan, are effective inducers of cytokine secretion by monocytes. Monocyte-synthesized cytokines have also been demonstrated to regulate monocyte cytokine synthesis via autoregulation. In particular, IL-1α, IL-1β, TNF-α, TGF-β, IFN-γ, GM-CSF and IL-3 have all been shown to stimulate some aspect of monocyte cytokine secretion, either acting alone or in combination with other stimuli. Conversely, IL-4 has potent antagonistic effects on the induction of monocyte activation, including both cytokine secretion and respiratory burst activity.

Activated macrophages produce and secrete various cytokines, including Interleukin-6 (IL-6), Interleukin-1 α and β (IL-1α, IL-1β), Tumor Necrosis Factor α, (TNF-α), Interleukin-8 (IL-8), Macrophage Inhibitory Peptide-1α (MIP-1α), Macrophage Inhibitory Peptide-1β, MIP-1β, Interleukin-12 (IL-12) and growth regulatory protein (GRO). Activation may thus be determined by measuring the secretion of one or more of these cytokines, or by analyzing levels of transcription of mRNA for one or more of these cytokines. Moreover, macrophages can be obtained and cultured *in vitro* (after activation either *in vitro* or *in vivo),* and activation can be determined by observing effective phagocytosis of microbe organisms and/or production of various cytokines. Methods of measuring the production and release of reactive oxygen species are well-known in the art.

IL-12, which is synthesized by macrophages in response to certain stimuli, is believed to be an initiation cytokine for cellular immunity (Scott, P., *Science* 260:496, 1993; Romagnini, S., *Immunol. Today* 13:379, 1992; Locksley, R.M., *Proc*, *Natl. Acad. Sci. USA* 90:5970, 1993). Monocytes/macrophages release IL-12 in response to various pathogens, stimulating natural killer cells (NK) and uncommitted T cells to secrete IFN-γ (Trinchierei, G., *Immunol. Today* 14:335, 1993). IL-12 also increases the cytolytic activity of T cells and NK cells (Gately et al., *Int. Immunol.* 6:57, 1994), and has been shown to play an important role in a number of infectious diseases (Gazzinelli et al., *Proc. Natl. Acad. Sci. USA* 90:6115, 1993; Heinzel et al., *J. Exp. Med.* 177:1505, 1993; Hsieh et al., *Science* 260:547, 1993; Tripp et al., *Proc. Natl. Acad. Sci. USA* 90:3725, 1993; Sypek et al., *J. Exp. Med.* 177:1797, 1993).

In other studies, IL-12 exhibited anti-tumor and anti-metastatic activities (Brunda et al., *J. Exp. Med.* 178:1223, 1993). Furthermore, IL-12 production has been found to be impaired in individuals infected with human immunodeficiency virus (HIV); the impaired ability to produce IL-12 is thought to play a role in the immunodeficiency that is the hallmark of HIV-related disease (Chehimi et al., *J. Exp. Med.* 179:1361, 1994). Levels of IL-12 can be assessed, for example, as described by Zhang et al. (*J. Clin. Invest.* 93:1733, 1994), or by enyme immunoassay or bioassay as described herein.

### Administration of CD40 binding proteins

The present invention provides methods of using therapeutic compositions comprising an effective amount of a CD40 binding protein and a suitable diluent and carrier, and methods for regulating an immune or inflammatory response. The use of CD40 binding proteins in conjunction with soluble cytokine receptors or cytokines, or other immunoregulatory molecules is also contemplated. For example, CD40 binding proteins can be used in conjunction with factors that are known to activate monocytes/macrophages, such as granulocyte-macrophage colony stimulating factor (GM-CSF), interferon-gamma (IFN-γ), and fusion proteins comprising GM-CSF such as those described in U.S. patent 5,073,627. The CD40 binding proteins and the factor(s) can either be combined in suitable solution, or can be administered simultaneously, sequentially or separately.

For therapeutic use, purified CD40 binding protein is administered to a patient, preferably a human, for treatment in a manner appropriate to the indication. Thus, for example, CD40 binding protein compositions administered to augment immune and/or inflammatory response can be given by bolus injection, continuous infusion, sustained release from implants, or other suitable technique. Typically, a therapeutic agent will be administered in the form of a composition comprising purified CD40 binding protein in conjunction with physiologically acceptable carriers, excipients or diluents. Such carriers will be nontoxic to recipients at the dosages and concentrations employed.

Ordinarily, the preparation of such CD40 binding protein compositions entails combining the CD40 binding protein with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with conspecific serum albumin are exemplary appropriate diluents. Preferably, product is formulated as a lyophilizate using appropriate excipient solutions (e.g., sucrose) as diluents.

Appropriate dosages can be determined in trials, first in an appropriate animal model, and subsequently in the species to be treated. The amount and frequency of administration will depend, of course, on such factors as the nature and severity of the indication being treated, the desired response, the condition of the individual being treated, and so forth. The appropriate dosages are within the range of about 10 ng/kg/day to about 100 µg/kg/day, alone or in combination with other immune response-regulating agents. Preferably a dose of 100 ng/kg/day to about 1000 ng/kg/day for about 1-20 days can be expected to induce an appropriate biological effect. Alternatively, bolus injections of from about 1 µg/kg/day to about 100 µg/kg/day can be given at approximately 4-day intervals to exert effects via stimulation of immune and/or inflammatory responses.

The relevant disclosures of all references cited herein are specifically incorporated by reference. The following examples are intended to illustrate particular embodiments, and not limit the scope, of the invention.

### Example 1

This example demonstrates that CD40 ligand is involved in T cell-dependent regulation of IL-12 production by splenic antigen presenting cells (APC). In this system, it was presumed that anti-CD3 would activate splenic T cells, and allow them to regulate the production of IL-12 by the splenic APC.

Unfractionated, non-irradiated splenocytes from naive C57BL/6 mice were cultured at 4 x 10⁵ cells/well in 96-well plates coated with antibody to CD3 (333 ng/well) in the presence or absence of 10 µg/well of anti-CD40L (MR1; available from PharMingen, San Diego, CA). Supernatants were removed after 20 hours and tested for the presence of various cytokines. IFN-γ levels were determined by two site ELISA using commercially available monoclonal antibody pairs (PharMingen, San Diego, CA) and performed according to a protocol supplied by PharMingen. The ELISA for IFN-γ was sensitive to 100 pg/ml of IFN-γ, using as a standard murine IFN-γ from Genzyme (Cambridge, MA). The assay for IL-12 was performed as described in Kennedy et al., *Eur. J. Immunol.* 24:2271 (1994). As shown in Table 1 below, IL-12 was produced in these cultures, and the inclusion of anti-CD40L inhibited IL-12 production by >90%.

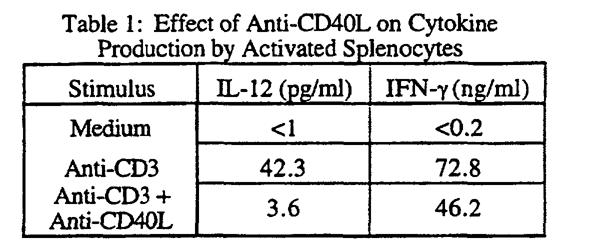

Anti-CD40L also partially inhibited the production of IFN-γ but not the production of IL-2. Furthermore, anti-CD40L did not enhance the production of IL-10 by the stimulated splenocytes. Thus, anti-CD40L did not inhibit the production of IL-12 production by inducing IL-10.

### Example 2

This example demonstrates that the production of IL-12 in an antigen-dependent system is dependent on CD40L. Varying numbers of a TH1 clone referred to as CD6 (specific for keyhole limpet hemocyanin; KLH) were stimulated in 96-well plates with 4 x 10⁵ irradiated syngeneic splenocytes and antigen (50 µg/ml KLH) in the presence or absence of anti-CD40L. Supernatants were collected at 20 hours and tested for IL-12 or IFN-γ as described above. Results are shown in Table 2 below.

**Table 2:**

| Dependence of Antigen-dependent Production of IL-12 Upon CD40L | | | | | |
|---|---|---|---|---|---|
| Cells/well | IL-12 (pg/ml) | | | IFN-γ(ng/ml) | |
| | Medium | Anti-CD40L^{a} | Anti-IFN-γ^{b} | Medium | Anti-CD40L |
| 20,000 | 22 | <2 | 25 | 30 | 24 |
| 10,000 | 24 | <2 | 22 | 23 | 17 |
| 5,000 | 16 | <2 | 15 | 13 | 8 |
| 2,500 | 10 | <2 | 8 | 7 | 5 |

| | | | | | |
|---|---|---|---|---|---|
| a: 10 µg/ml MR1, available from PharMingen, San Diego, CA. | | | | | |
| b: 1:500 (v/v) XMG1.2, available from PharMingen, San Diego, CA. | | | | | |

The inclusion of anti-CD40L inhibited the production of IL-12 in this antigen dependent system by >90%. In contrast, inclusion of neutralizing antibody to IFN-γ had no inhibitory effect on the production of IL-12. Despite its strong inhibitory effects on IL-12 production, anti-CD40L only partially inhibited the production of IFN-γ in these same cultures. IL-2 was not detected in any of the supernatants.

Similar results were observed when an H-2^{d}-specific, alloreactive T_{H}1 clone referred to as C3G9 was stimulated with irradiated C.B17 SCID splenocytes or adherent BALB/c peritoneal exudate cells (PEC). In both cases, the inclusion of anti-CD40L in these cultures inhibited the production of IL-12 by >90%, whereas inclusion of anti-IFN-γ had no inhibitory effect.

### Example 3

This example demonstrates that splenocytes from mice lacking a functional CD40L appear to be deficient in T cell dependent production of IL-12. Splenocytes were stimulated substantially as described in Example 1 above. Unfractionated, non-irradiated splenocytes from naive C57BL/6 X 129/J mice homozygous for disruption of the CD40 ligand gene (CD40 ligand knockout, or CD40L KO mice, described in USSN 08/184,422, filed January 20, 1994, now pending) or control B6 (C57BL/6) or F1 (B6x129) were cultured at 4 x 10⁵ cells/well in 96-well plates coated with antibody to CD3 (333 ng/well). Supernatants were removed after 20 hours and tested for the presence of various cytokines by either bioassay (IL-12 and IL-2, Figures 1A and 1B) or ELISA (IFN-γ and IL-10, Figures 1C and 1D). The IL-2 bioassay utilized the CTLL-2 cell line, as described in Kennedy et al., *supra.* The IL-10 ELISA was similar to the previously described IFN-γ ELISA, utilizing monoclonal antibodies and protocol from PharMingen. Purified IL-10 for use as a standard is available from Biosource International (Camarillo, CA)or Genzyme (Cambridge, MA). The results are presented in Figure 1, and are representative of three experiments.

IL-12 was produced by anti-CD3 stimulated splenocytes from B6 and F1 mice, but was not detected (<2 pg/ml) in supernatants from anti-CD3 stimulated CD40L KO splenocytes. In contrast, CD40L splenocytes had no apparent defect in terms of their ability to produce IL-2, IL-10 or IFN-γ although the level of IFN-γ (ng/ml) produced by the CD40L KO splenocytes was lower than that of the controls (Figure 1C). The amount of IL-10 produced by both the CD40L KO and the B6 control mice was consistently lower than that produced by the F1 control mice (Figure 1D). None of the cytokines was detected in cultures that lacked anti-CD3.

### Example 4

This example demonstrates that anti-IL-10 and a soluble, trimeric form of the CD40L (CD40LT) enhance T cell-dependent IL-12 production by CD40L KO splenocytes. Since IL-10, which inhibits IL-12 production, is also present in the cultures described above, duplicate cultures were set up as described above, in the presence of anti-CD3 alone, or with the inclusion of anti-IL-10 (2 µg/ml). Supernatant from control splenocytes stimulated with anti-CD3 in the presence of anti-IL-10 contained approximately four-fold higher levels of IL-12 than those generated in the absence of anti-IL-10 (Figure 2). Furthermore, low levels of IL-12 were detected in supernatants for CD40L KO splenocytes incubated with both anti-CD3 and anti-IL-10 (Figure 2).

The ability of CD40L KO splenocytes to produce IL-12 under these conditions in the presence or absence of CD40LT is shown in Table 3 below.

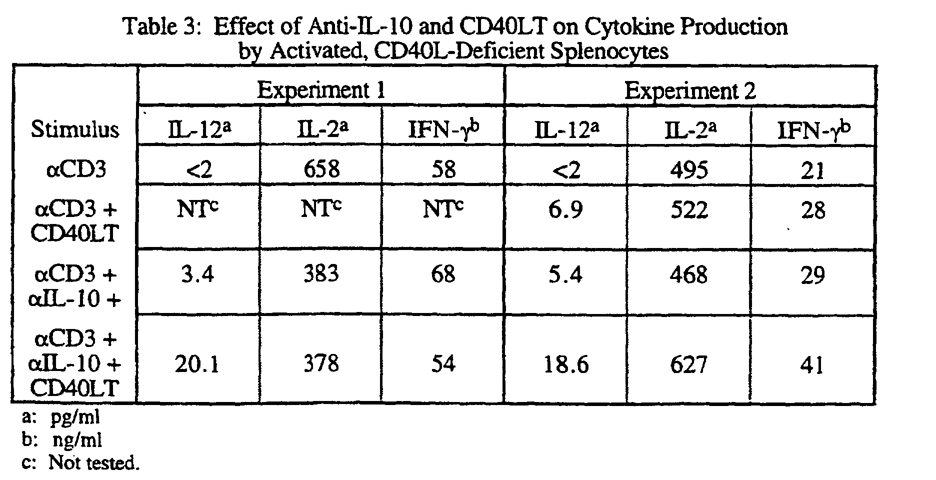

CD40L KO splenocytes stimulated with anti-CD3, anti-IL-10 and CD40L trimer (25 µg/ml) produced approximately 5-fold higher levels of IL-12 than the CD40L KO splenocytes stimulated with anti-CD3 + anti-IL-10 alone. CD40LT also induced low levels of IL-12 in cultures that contained anti-CD3 but not anti-IL-10. In contrast to its effects on the production of IL-12 by the anti-CD3 activated CD40L KO splenocytes, CD40LT did not consistently enhance the production of IL-2 or IFN-γ in these cultures. None of the cytokines was detected in cultures that lacked anti-CD3.

### Example 5

This example demonstrates that a soluble, trimeric form of the CD40L (CD40LT) enhances IL-12 production by human monocytes. Whole blood was obtained from two different donors, and monocytes were isolated by countercurrent elutriation as described in Alderson et al., *J. Exp. Med.* 178:669 (1993). The monocytes were cultured at 5 x 10⁵ cells/well, in 24-well plates (Costar Corp., Cambridge, MA), either in medium alone, or in the presence CD40LT (1µg/ml), IFN-γ (10 ng/ml), or GM-CSF (10 ng/ml) alone or in combination (CD40LT + IFN-γ or CD40LT + GM-CSF). A neutralizing monoclonal antibody to CD40L (10 µg/ml) was included to determine whether the costimulatory effect was specific. After 24 hours of culture, supernatants were recovered and tested for the presence of IL-12 using a commercially available EIA that detects heterodimeric IL-12 (R&D Systems, Minneapolis, MN). Results are shown in Table 4 below.

**Table 4:**

| Effect of CD40LT on IL-12 Production by Human Monocytes | | |
|---|---|---|
| Stimulus | Donor 1 | Donor 2 |
| None | <7.8 | <7.8 |
| CD40LT | <7.8 | <7.8 |
| IFN-γ | 105.6 | 53.2 |
| GM-CSF | <7.8 | <7.8 |
| IFN-γ + CD40LT | 495.3 | 146.2 |
| GM-CSF + CD40LT | <7.8 | <7.8 |
| IFN-γ + CD40LT + anti CD40L | 107.5 | 58.3 |

The addition of CD40LT and IFN-γ to culture medium resulted in enhanced production of IL-12 (four to five fold), as compared to IFN-γ alone. This enhancement was specifically due to CD40LT, as demonstrated by the ability to block the effect with a monoclonal antibody that specifically binds CD40L and inhibits binding of CD40 to its receptor, CD40. GM-CSF and CD40LT did not stimulate secretion of IL-12 in these experiments, alone or in combination, a marked difference from the ability of either GM-CSF or IFN-γ to costimulate production of other cytokines by monocytes stimulated with membrane-bound CD40L (Alderson et al., *supra).* These results demonstrate that soluble CD40L is a potent costimulus for IL-12 secretion by human monocytes.

### Example 6

This example demonstrates that CD40L KO mice exhibit severe T cell anergy. CD40L KO mice and control C57BL/6 X 129/J F1 hybrids (F1) were tested for delayed-type hypersensitivity (DTH) reaction to purified protein derivative (PPD) essentially according to the method of Gray and Jennings (*Ann. Rev. Tuberculosis* 72:171, 1955), as described in Van Buren, et al., *Transplantation* 40:694 (1985).

Mice (CD40L KO or controls) were immunized subcutaneously with 200 µl complete Freund's adjuvant (CFA; H37Ra). Three weeks later, the immunized mice and a group of unimmunized controls were challenged by intracutaneous injection of 2 µg of PPD in a volume of 50 µl, into the rear footpad. An equal volume of normal saline was injected into the contralateral footpad at the same time.

After 48 hours, footpad thickness was measured with a micrometer; results were expressed as the difference in the amount of swelling between PPD challenged and saline challenged footpads, and were compared to the swelling observed with non-immunized animals challenged with PPD. Results are shown in Figure 3.

The results demonstrate that, as a group, CD40L-deficient mice are severely impaired in their ability to mount a DTH response to antigen (Figure 3B), although there was some variation in response among the individual CD40L KO mice tested (Figure 3A). These results confirm that CD40L is critical for the development of a cellular immune response to antigen.

### Example 7

This example demonstrates that CD40 ligand is important in the generation of a cell-mediated immune response to *Leishmania.* The course of *L. major* infection in different inbred mouse strains is determined by differential development of T_{H}1 or T_{H}2 CD4+ T lymphocytes. Expansion of IL-4 secreting T_{H}2 cells renders BALB/c mice susceptible to disease and parasite dissemination. In contrast, expansion of IFN-γ producing T_{H}1 cells enables resistant mouse strains, including C57BL/6 and 129/J, to establish protective immunity.

CD40L KO mice or control mice (129/J, C57BL/6) were infected with 2x10⁵ *L*. *major* by injection in the hind footpad. Disease progression was determined by observing the physical symptoms, and by measuring the footpad thickness of the infected foot as compared to the uninfected, contralateral foot. CD40L KO mice developed large lesions on the infected hind footpad within 4-6 weeks after promastigote inoculation. By contrast, control mice resisted progression of *L. major* infection. The mean changes in footpad size are presented in Figure 4.

Lymphocytes from the spleen and draining lymph nodes (LNC) of infected animals were stimulated *in vitro* with immobilized anti-CD3 or soluble *Leishmania* antigen, and cytokine secretion was assessed. IFN-γ levels were determined by two site ELISA as described previously. IL-4 levels were determined by a similar method, using antibodies and a protocol from PharMingen, using a recombinant murine IL4 prepared at Immunex Corporation (Seattle, WA) as a standard. Results are shown in Table 5.

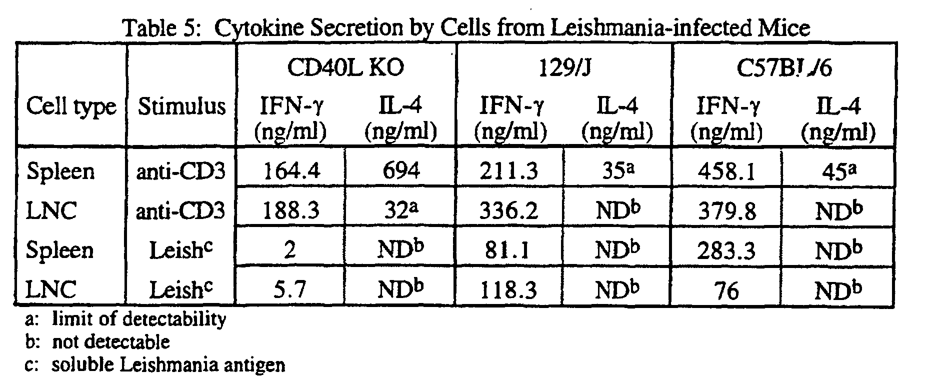

Cells from CD40L KO mice produced significantly less IFN-γ and more IL-4 than cells from control mice, suggesting that CD40L KO mice exhibit a defect in their ability to mount a T_{H}1 response to *Leishmania*. These results demonstrate that CD40L KO mice are susceptible to *L. major* despite their resistant background.

### Example 8

This example demonstrates that soluble, trimeric CD40 ligand ameliorates the course of leishmaniasis in susceptible mice. CD40L KO mice, susceptible BALB/c mice and control C57BL/6 X 129/J F1 (F1) hybrids were infected with *L. major* as described above. A soluble, trimeric form of recombinant CD40 ligand (CD40LT) was administered to the mice (50 µg/day) daily for two weeks, beginning on day 0, the day of infection. Disease progression was monitored by measuring footpad thickness as described previously, and by observing the symptoms of disease. Results are shown in Figure 5. CD40 ligand diminished the physiological severity of leishmaniasis in both CD40L KO and BALB/c mice.

### Example 9

This example demonstrates that soluble, trimeric CD40 ligand controls or ameliorates infection of susceptible mice by *Pneumocystis.* CD40L KO mice were maintained in a specific pathogen-free environment. CD40LT was administered to one group of mice (n=12) three times per week for twelve weeks (50 µg/day), beginning on the second day after birth. A second group of mice (n=12) were given CD40LT three times per week for five weeks (50 µg/day), beginning at the sixteenth week after birth. Control mice (n=34) were given no CD40LT. Results are shown in Figure 6.

The control mice began dying at about five months of age; all of them were dead by about 11 months. Necropsies were performed on the dead animals, and histologic studies carried out to determine the cause of death. The only pathogenic/opportunistic agent identified was *Pneumocystis;* the lungs of the dead mice demonstrated characteristic effects of *Pneumocystis* infection by silver stain. In contrast, mice given CD40LT, either for 12 weeks beginning soon after birth or for 5 weeks starting at 16 weeks of age survived considerably longer; the earliest deaths occurred at about ten months, and the vast majority of the mice were alive and apparently healthy past one year of age. Thus, CD40L KO mice are a useful animal model of *Pneumocystis* infection; this opportunistic organisms appears to be present in mice as 'normal' flora, but disease is held in check by an intact cell-mediated immune system. CD40 ligand will be useful in controlling *Pneumocystis* infection in susceptible individuals, and will similarly be useful in controlling other diseases in individuals having a depressed cellular immune response.

## Claims

1. The use of a CD40 binding protein in the preparation of a composition for stimulating a cell-mediated, T_{H}1 immune response in a mammal infected with a pathogenic or opportunistic organism.

2. The use according to claim 1 wherein the pathogenic or opportunistic organism is selected from the group consisting *of Trypanosoma, Salmonella, Pneumocystis, Toxoplasma, Listeria, Mycobacteria* and *Leishmania.*

3. The use of a CD40 binding protein in the preparation of a composition for stimulating macrophages to secrete Interleukin-12 in a mammal in need thereof.

4. The use according to claim 3 wherein the mammal is infected with human immunodeficiency virus.

5. The use of a CD40 binding protein in the preparation of a composition for administration to a mammal afflicted with a depressed cellular immune response in order to stimulate a cellular immune response.

6. The use of claim 5 wherein the mammal is infected with human immunodeficiency virus.

7. The use of a CD40 binding protein in the preparation of a composition for stimulating anti-tumour activity in macrophages in a mammal afflicted with a tumour.

8. The use according to any preceding claim wherein the CD40 binding protein is selected from the group consisting of CD40 ligand, soluble oligomeric CD40-L, monoclonal antibodies that specifically bind CD40, and combinations thereof.

## Patentansprüche

1. Verwendung von CD40 Bindungsproteinen für die Herstellung einer Zusammensetzung zur Stimulierung einer zellvermittelten T_{H}1 Immunantwort im Säugetier, welches mit pathogenen oder nützlichen Mikroorganismen infiziert ist.

2. Verwendung nach Anspruch 1, **dadurch gekennzeichnet, daß** die pathogenen oder nützlichen Organismen aus der Gruppe bestehend aus *Trypanosoma, Salmonella, Pneumosystis*, *Toxoplasma, Listeria, Mycobakteria und Leishmania* ausgewählt sind.

3. Verwendung von CD40 Bindungsprotein für die Herstellung einer Zusammensetzung zur Stimulierung von Makrophagen, die in Säugetieren das benötigte Interleukin-12 ausscheiden.

4. Verwendung nach Anspruch 3, **dadurch gekennzeichnet, dass** das Säugetier mit dem menschlichen Immunschwäche Virus infiziert ist.

5. Verwendung von CD40 Bindungsprotein für die Herstellung einer Zusammensetzung zur Verabreichung an Säugetiere, die an unterdrückter zellulärer Immunantwort leiden, um eine zelluläre Immunantwort zu stimulieren.

6. Verwendung nach Anspruch 5, **dadurch gekennzeichnet, dass** das Säugetier mit dem menschlichen Immunschwäche-Virus infiziert ist.

7. Verwendung von CD40 Bindungsprotein für die Herstellung einer Zusammensetzung zur Stimulierung der Anti-Tumor-Aktivität der Makrophagen in einem Tumor-erkrankten Säugetier.

8. Verwendung gemäß einem der vorausgehenden Ansprüche, **dadurch gekennzeichnet, dass** das CD40 Bindungsprotein aus der Gruppe bestehend aus CD40- Liganden, löslichen Oligomeren des CD40-L, monoklonalen Antikörpern, die CD40 spezifisch binden, und deren Kombinationen ausgewählt ist.

## Revendications

1. Utilisation d'une protéine de fixation de CD40 dans la préparation d'une composition pour la stimulation d'une réponse immunitaire T_{H}1 à médiation cellulaire chez un mammifère infecté par un organisme pathogène ou opportuniste.

2. Utilisation selon la revendication 1, dans laquelle l'organisme pathogène ou opportuniste est choisi dans le groupe constitué par *Trypanosoma, Salmonella, Pneumocystis, Toxoplasma, Listeria, Mycobacteria* et *Leishmania.*

3. Utilisation d'une protéine de fixation de CD40 dans la préparation d'une composition pour la stimulation de la sécrétion d'interleukine -12 par les macrophages chez un mammifère en ayant besoin.

4. Utilisation selon la revendication 3, dans laquelle le mammifère est infecté par un virus de l'immunodéficience humaine.

5. Utilisation d'une protéine de fixation de CD40 dans la préparation d'une composition à administrer à un mammifère souffrant d'une déficience de la réponse immunitaire cellulaire afin de stimuler une réponse immunitaire cellulaire.

6. Utilisation selon la revendication 5, dans laquelle le mammifère est infecté par un virus de l'immunodéficience humaine.

7. Utilisation d'une protéine de fixation de CD40 dans la préparation d'une composition pour la stimulation d'une activité antitumorale dans les macrophages chez un mammifère souffrant d'une tumeur.

8. Utilisation selon l'une quelconque des revendications précédentes, dans laquelle la protéine de fixation de CD40 est choisie dans le groupe constitué par le ligande de CD40, CD40-L oligomère soluble, des anticorps monoclonaux qui se fixent spécifiquement à CD40 et leurs combinaisons.
